(19) Europäisches Patentamt
European Patent Office
Office européen des brevets

(11) EP 0 712 856 B1

(12) **FASCICULE DE BREVET EUROPEEN**

(45) Date de publication et mention de la délivrance du brevet:
**28.01.1998 Bulletin 1998/05**

(51) Int. Cl.$^6$: **C07F 7/08**, C07F 7/21, C08G 77/388

(21) Numéro de dépôt: **95402308.1**

(22) Date de dépôt: **16.10.1995**

(54) **Nouveaux filtres solaires, compositions cosmétiques photoprotectrices les contenant et utilisations**

Sonnenfilter, diese enthaltende kosmetische Sonnenschutzzusammensetzungen und deren Verwendung

Sunscreens, cosmetic sunscreen compositions and use thereof

(84) Etats contractants désignés:
**AT BE CH DE DK ES FR GB GR IE IT LI LU MC NL PT SE**

(30) Priorité: **17.11.1994 FR 9413770**

(43) Date de publication de la demande:
**22.05.1996 Bulletin 1996/21**

(73) Titulaire: **L'OREAL**
**75008 Paris (FR)**

(72) Inventeurs:
- **Richard, Hervé**
  **F-93420 Villepinte (FR)**
- **Leduc, Madeleine,**
  **Résidence Les Chèvrefeuilles**
  **F-75011 Paris (FR)**
- **Lagrange, Alain**
  **F-77700 Couvray (FR)**

(74) Mandataire:
**Andral, Christophe André Louis**
**L'OREAL**
**Centre de Recherche Charles Zviak**
**Département Propriété Industrielle**
**90, rue du Général Roguet**
**92583 Clichy Cedex (FR)**

(56) Documents cités:
**WO-A-94/06404** **DE-A- 1 907 403**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

EP 0 712 856 B1

Printed by Xerox (UK) Business Services
2.15.8/3.4

## Description

La présente invention concerne de nouveaux composés du type diorganosiloxanes, à chaines courtes, linéaires ou cycliques, ou du type triorganosilanes, présentant pour caractéristique commune d'avoir tous au moins un motif filtrant dérivé d'un oxanilide à fonction alkylène ou alkylèneoxy, ces composés étant plus particulièrement utilisables à titre de filtres organiques solaires dans des compositions cosmétiques destinées à la protection de la peau et des cheveux contre le rayonnement ultraviolet. L'invention concerne également l'utilisation desdits composés dans l'application cosmétique susmentionnée, ainsi que les compositions cosmétiques à propriétés améliorées les contenant.

On sait que les radiations lumineuses de longueurs d'onde comprises entre 280 nm et 400 nm permettent le brunissement de l'épiderme humain, et que les rayons de longueurs d'onde plus particulièrement comprises entre 280 et 320 nm, connus sous la dénomination d'UV-B, provoquent des érythèmes et des brûlures cutanées qui peuvent nuire au développement du bronzage naturel ; il convient donc de filtrer ce rayonnement UV-B.

On sait également que les rayons UV-A, de longueurs d'onde comprises entre 320 et 400 nm, qui provoquent le brunissement de la peau, sont susceptibles d'induire une altération de celle-ci, notamment dans le cas d'une peau sensible ou d'une peau continuellement exposée au rayonnement solaire. Les rayons UV-A provoquent en particulier une perte d'élasticité de la peau et l'apparition de rides conduisant à un vieillissement cutané prématuré. Ils favorisent le déclenchement de la réaction érythémateuse ou amplifient cette réaction chez certains sujets et peuvent même être à l'origine de réactions phototoxiques ou photo-allergiques. Il est donc souhaitable de filtrer aussi le rayonnement UV-A.

De nombreux composés destinées à la photoprotection (UV-A et/ou UV-B) de la peau ont été proposées à ce jour.

La plupart d'entre eux sont des composés aromatiques présentant une absorption des rayons UV dans la zone comprise entre 280 et 315 nm, ou dans la zone comprise entre 315 et 400 nm ou bien encore dans l'ensemble de ces deux zones. Ils sont le plus souvent formulés dans des compositions antisolaires qui se présentent sous la forme d'une émulsion de type huile-dans-eau (c'est à dire un support cosmétiquement acceptable constitué d'une phase continue dispersante aqueuse et d'une phase discontinue dispersée huileuse) et qui contiennent donc, à des concentrations diverses, un ou plusieurs filtres organiques classiques à fonction aromatique, lipophiles et/ou hydrophiles, capables d'absorber selectivement les rayonnements UV nocifs, ces filtres (et leurs quantités) étant sélectionnés en fonction de l'indice de protection recherché (l'indice de protection (IP) s'exprimant mathématiquement par le rapport du temps d'irradiation nécessaire pour atteindre le seuil érythématogène avec le filtre UV au temps nécessaire pour atteindre le seuil érythématogène sans filtre UV).

Outre leur pouvoir filtrant intrinsèque, ces composés à activité anti-UV doivent également présenter de bonnes propriétés cosmétiques dans les compositions qui les contiennent, une bonne solubilité dans les solvants usuels et en particulier les corps gras tels que les huiles et les graisses, ainsi qu'une bonne résistance à l'eau et à la transpiration (rémanence). Il est bien entendu également souhaitable que ces substances filtrantes ne pénètrent pas dans la peau, et soient non toxiques.

Parmi tous les composés aromatiques qui ont été préconisés à cet effet, nombre d'entre eux ne présentent pas toutes les propriétés requises pour une utilisation convenable comme filtres UV dans les compositions antisolaires. En particulier, leur pouvoir filtrant intrinsèque peut être insuffisant, et de ce fait, afin d'obtenir des propriétés filtrantes satisfaisantes, il est souvent nécessaire de mettre en oeuvre des quantités relativement importantes de produits, ce qui se traduit par de mauvaises propriétés cosmétiques au niveau des formulations finales qui les contiennent ; en outre, leur solubilité dans les différents type de formulations utilisés en matière de protection solaire n'est pas toujours suffisamment bonne (liposolubilité notamment) ; elles peuvent par ailleurs ne pas posséder une stabilité suffisante à la lumière (photostabilité) ; et elles peuvent enfin également présenter une mauvaise résistance à l'eau et à la sueur.

Il est connu du document WO94/06404 des compositions comprenant du 4-(tert.-butyl) 4'-méthoxy dibenzoylméthane et un polymère filtrant du type silicone benzotriazole.

La présente invention vise à proposer une nouvelle famille de composés, plus particulièrement utilisables à titre de filtres solaires pour compositions cosmétiques à effets anti-UV, et ne présentant pas les inconvénients ci-dessus.

Ainsi, selon la présente invention, il a été trouvé qu'en venant fixer par greffage, notamment selon une réaction d'hydrosylilation, un ou plusieurs motifs filtrants particuliers, à savoir des dérivés d'oxanilides, sur une chaine silicone convenablement selectionnée, linéaire ou cyclique, ou sur un silane convenablement sélectionné, et ceci par l'intermédiaire d'un chainon de raccordement spécifique de type alkylène ou alkylèneoxy, il était possible d'aboutir à de nouveaux composés à propriétés remarquables, ces nouveaux composés présentant en particulier de très bonnes propriétés filtrantes, soit dans l'UV-A soit dans l'UV-B selon leur structure chimique, une très bonne solubilité dans les solvants organiques usuels et notamment les corps gras tels que les huiles, une excellente photostabilité, ainsi que d'excellentes propriétés cosmétiques, les rendant particulièrement appropriés à une utilisation comme filtres solaires dans des, ou pour la préparation de, compositions cosmétiques destinées à la protection de la peau et/ou des cheveux contre le rayonnement ultraviolet.

Cette découverte est à la base de la présente invention.

La présente invention a ainsi pour premier objet de nouveaux composés qui sont caractérisés par le fait qu'ils

répondent à l'une des formules (1) à (3) suivantes :

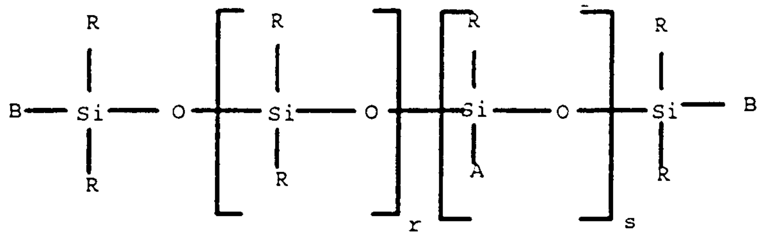

(1)

ou

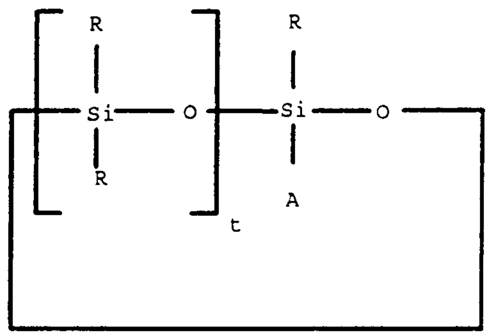

(2)

ou

$$A\text{-}Si(R')_3 \quad (3)$$

formules (1) à (3) dans lesquelles :

- R, identiques ou différents, sont choisis parmi les radicaux alkyles en $C_1$-$C_{10}$, phényle et trifluoro-3,3,3 propyle, au moins 80% en nombre des radicaux R étant méthyle,
- B, identiques ou différents, sont choisis parmi les radicaux R ci-dessus et le radical A défini ci-après,
- R', identiques ou différents, sont choisis parmi les radicaux alkyles en $C_1$-$C_8$, ou phényle,
- r est un nombre entier compris entre 0 et 50 inclusivement, et s est un nombre entier égal à 0 ou 1, avec la condition que si s est nul alors au moins l'un des deux symboles B désigne A,
- t est un nombre entier compris entre 2 et 10 inclusivement,
- et le symbole A désigne un radical lié directement à un atome de silicium, et qui répond à la formule (4) suivante :

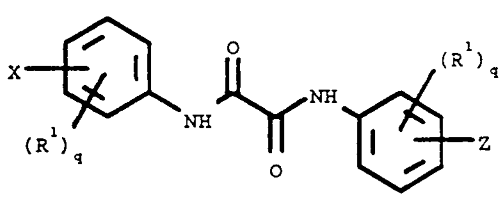

(4)

formule (4) dans laquelle :

* **X** représente un radical divalent -Y- de formule (5) suivante :

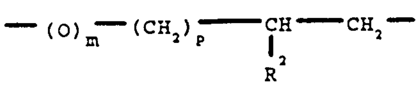

(5)

formule (5) dans laquelle :

- $R^2$ désigne un atome d'hydrogène ou un radical alkyle en $C_1$-$C_4$,
- p est un nombre entier compris entre 0 et 10 inclusivement,
- m est 0 ou 1
- le groupement $-CH_2-$ terminal est directement lié à un atome de silicium ;

* **Z** représente un atome d'hydrogène ou un radical divalent -Y-,

* **q** est un nombre entier compris inclusivement entre 0 et 3,

* $\mathbf{R^1}$, identiques ou différents, sont choisis parmi les radicaux alkyles en $C_1$-$C_8$ ou les radicaux alcoxy en $C_1$-$C_6$, étant entendu que dans ce dernier cas deux $R^1$ adjacents ($q \geq 2$) peuvent former ensemble un groupement alkylidène dioxy dans lequel le groupe alkylidène contient 1 ou 2 atomes de carbone.

Dans les formules (1) à (3) ci-dessus, A représente donc le groupement dérivé de l'oxanilide qui, après fixation sur la chaine courte siliconée de départ ou sur le silane de départ, confère aux composés de type diorganosiloxanes linéaires (formule (1)) ou cycliques (formule (2)) ou de type triorganosilanes (formule (3)) des propriétés absorbantes vis à vis de l'ultraviolet dans une zone de longueur d'onde pouvant aller de 280 à 400 nm. Comme indiqué précédemment, et comme cela ressort de la définition de la formule (4) donnée ci-dessus, ce groupement présente nécessairement au moins une fonction alkylène ou alkylèneoxy correspondant au chainon qui assure l'accrochage de l'oxanilide à la chaine silicone ou au silane. L'un des avantages des composés selon l'invention est que l'on peut obtenir, selon la nature et/ou la position des différents groupements portés sur le motif filtrant A, des filtres soit purement UV-A ou au contraire purement UV-B, et ceci avec des coefficients d'extinction particulièrement élevés.

Comme cela ressort des définitions données ci-avant, l'accrochage d'un chainon $-(O)_m-(CH_2)_p-CH(R^2)-CH_2-$ (c'est à dire d'un radical divalent -Y- de formule 5) sur le motif dérivé de l'oxanilide, qui assure donc le raccordement dudit motif à l'atome de silicium de la chaine siliconée ou du silane, peut, selon la présente invention, se faire à l'une quelconque des positions susceptibles d'être occupées par les symboles X ou Z sur les deux noyaux aromatiques de l'oxanilide, la partie terminale $-(O)_m-$ dudit chainon venant se fixer sur le motif dérivé de l'oxanilide et sa partie terminale $-CH_2-$ sur un atome de silicium de la chaine siliconée ou du silane. Il convient de souligner que le motif filtrant dérivé de l'oxanilide peut donc posséder soit un seul chainon de raccordement (cas où Z représente l'hydrogène), soit au contraire deux chainons de raccordement (cas où Z représente aussi un radical divalent -Y-) et être ainsi relié à deux chaines siliconées ou à deux motifs sylilés différents.

De préférence, selon la présente invention, le symbole X se trouve en position ortho ou para du noyau aromatique qui le porte.

De même, dans le cas où Z représente un deuxième chainon de raccordement -Y-, celui-ci est de préférence en position ortho ou para du noyau aromatique qui le porte.

Dans les formules (1) à (3) ci-dessus, les radicaux alkyle peuvent être linéaires ou ramifiés et choisis notamment au sein des radicaux méthyle, éthyle, n-propyle, isopropyle, n-butyle, isobutyle, ter.-butyle, n-amyle, isoamyle, néopentyle, n-hexyle, n-heptyle, n-octyle, éthyl-2 hexyle et ter.-octyle. Les radicaux alkyle R, R' et B préférés selon l'invention sont les radicaux méthyle, éthyle, propyle, n-butyle, n-octyle et éthyl-2 hexyle. Encore plus préférentiellement, les radicaux R, R' et B sont tous des radicaux méthyle. Les radicaux alcoxy peuvent être, quant à eux, choisis notamment parmi les radicaux méthoxy, éthoxy, n-propoxy, isopropoxy, n-butoxy et isobutoxy.

Parmi les composés de formules (1) à (3) ci-dessus, on préfère mettre en oeuvre ceux répondant à la formule (1) ou à la formule (2), c'est à dire des diorganosiloxanes à chaine courte, linéaire ou cyclique.

Parmi les diorganosiloxanes linéaires ou cycliques rentrant dans le cadre de la présente invention, on préfère plus particulièrement les dérivés statistiques ou bien définis à blocs présentant au moins l'une, et encore plus préférentiellement l'ensemble, des caractéristiques suivantes :

- R est alkyle et encore plus préférentiellement méthyle,
- B est alkyle et encore plus préférentiellement méthyle (cas des composés linéaires de formule (1)),

- r est compris entre 0 et 3 inclusivement (cas des composés linéaires de formule (1)),
- t est compris entre 2 et 4 inclusivement (cas des composés cycliques de formule (2)),
- q est nul ou égal à 1
- $R^1$ est H (q=0) ou méthoxy (q≠0),
- p est nul ou égal à 1,
- $R^2$ est H ou méthyle.

Pour préparer les filtres siliconés de formule (1) et (2), on peut procéder classiquement en mettant en oeuvre une réaction d'hydrosylilation

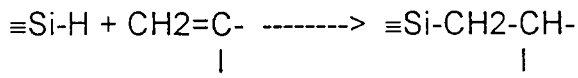

à partir de la silicone correspondante dans laquelle, par exemple, tous les radicaux A sont des atomes d'hydrogène. Cette silicone de départ est dénommée par la suite dérivé à SiH ; les groupes SiH peuvent être présents dans la chaîne et/ou aux extrémités de la chaîne silicone. Ces dérivés à SiH sont des produits bien connus dans l'industrie des silicones et sont généralement disponibles dans le commerce. Ils sont par exemple décrits dans les brevets américains US-A- 3 220 972, US-A- 3 697 473 et US-A- 4 340 709.

Ce dérivé à SiH peut être donc représenté, selon les cas, soit par la formule (1bis) suivante :

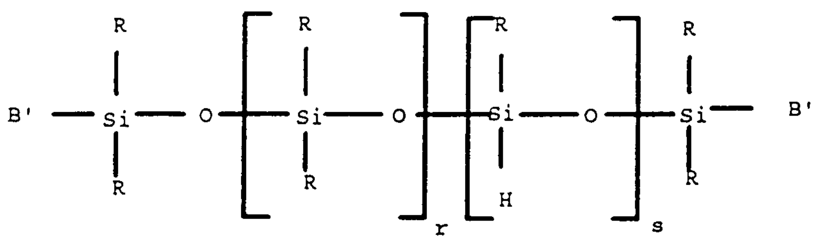

(1bis)

dans laquelle R, r et s ont la signification donnée ci-dessus pour la formule (1) et les radicaux B', identiques ou différents, sont choisis parmi les radicaux R et un atomé d'hydrogène, étant entendu que si s=0 alors l'un, et seulement l'un seul, des radicaux B' doit représenter un atome d'hydrogène,
soit par la formule (2bis) suivante :

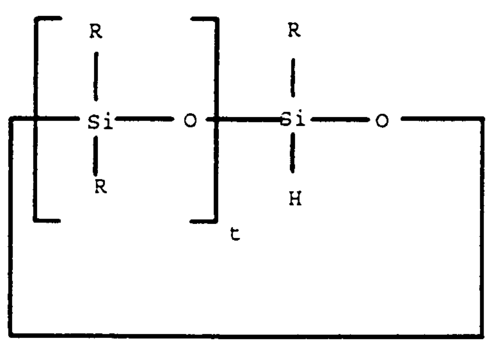

(2bis)

dans laquelle R et t ont la signification donnée ci-dessus pour la formule (2).

Sur ce dérivé à SiH de formule (1bis) ou (2bis), on effectue donc une réaction d'hydrosilylation classique, opérée en présence d'une quantité catalytiquement efficace d'un catalyseur au platine, sur un dérivé organique d'oxanilide choisi parmi ceux de formule (4bis) suivante :

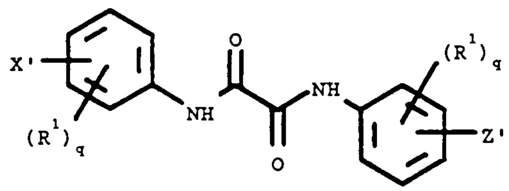

(4bis)

dans laquelle $R^1$ et q ont la même signification que celle donnée ci-avant pour la formule (4), et X' , au lieu de représenter le radical divalent -Y- de formule (5) définie ci-avant, représente ici le radical monovalent homologue insaturé correspondant -Y', de formule (5bis) suivante :

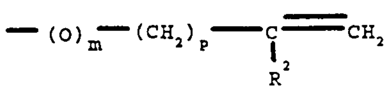

(5bis)

dans laquelle $R^2$, m et p ont la même signification qu'à la formule (5), et Z' désigne ou un monoradical -Y' tel que défini ci-dessus. Parmi les composés de formule (4bis) ci-dessus rentrant particulièrement bien dans le cadre de la présente invention, on peut notamment citer les :

a) N,N'-Bis-[4-(2-méthyl-allyloxy)-phényl]-oxalamide
b) N,N'-Bis-[2-(2-méthyl-allyloxy)-phényl]-oxalamide
c) N,N'-Bis-(2-allyloxy-5-méthoxy-phényl)-oxalamide

Des procédés convenant à la préparation des produits de formule (4bis) ci-dessus sont notamment décrits dans les brevets FR-B- 1 506 632 et FR-B- 1 516 276. En suivant l'enseignement de ces brevets, les produits peuvent ainsi être obtenus selon des méthodes connues suivantes :

1/ <u>Pour les dérivés symétriques</u> : par réaction, dans un premier stade, de l'acide oxalique ou de ses esters sur des amines aromatiques appropriées selon le schéma réactionnel (6) suivant :

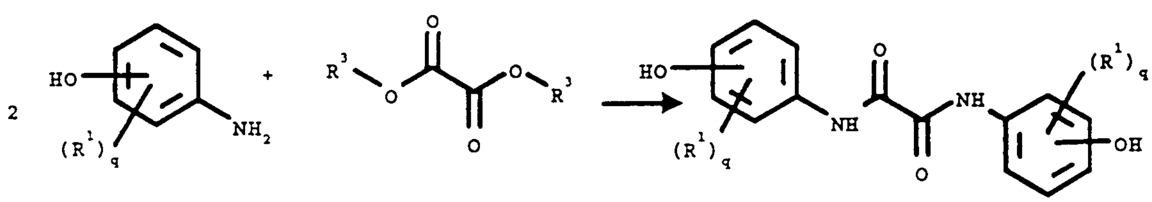

(6)

schéma dans lequel $R^1$ et q ont la même signification qu'à la formule (4), et $R^3$ représente un atome d'hydrogène, un radical méthyle ou éthyle.

Puis, dans un deuxième stade, les dérivés ainsi obtenus peuvent ensuite être alkylés par des halogénures de formule (7) suivante :

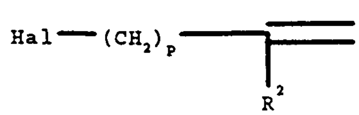

(7)

dans laquelle $R^2$ et p ont la même signification qu'à la formule (5) et Hal est un atome d'halogène choisi parmi le chlore et le brome,
ce par quoi l'on obtient finalement les dérivés de formule (4bis) désirés dans lesquels Z' représente -Y'.

2/ <u>Pour les dérivés asymmétriques</u>, la réaction conduisant aux dérivés décrits dans le schéma (6) se fait en deux

temps : (i) réaction de l'oxatate de diméthyle ou de diéthyle sur un équivalent d'amine aromatique contenant un hydroxyle, puis (ii) réaction du produit issu de (i) avec un second équivalent d'amine ne contenant pas d'hydroxyle. Le produit de réaction issu de (ii) est ensuite alkylé avec des halogénures de formule (7) donnée ci-avant, ce par quoi l'on obtient finalement les dérivés de formule (4bis) désirés dans lesquels Z' = H.

3/ Les dérivés de formule (4bis) pour lesquels le m de la formule (5bis) est égal à 0 peuvent être obtenus à partir des dérivés de formule (4bis) correspondants pour lesquels m =1, par transposition de Claisen suivie d'une alkylation de l'hydroxyle libre par un halogénure d'alkyle en $C_1$-$C_8$.

Les catalyseurs au platine utilisés pour réaliser la réaction d'hydrosylilation entre les composés de formule (1bis) ou (2bis) ci-dessus avec le composé de formule (4bis) ci-dessus sont bien connus et amplement décrits dans la littérature. On peut en particulier citer les complexes du platine et d'un produit organique décrit dans les brevets américains US-A- 3 159 601, US-A- 3 159 602, US-A- 3 220 972 et les demandes de brevets européens EP-A- 0 057 459, EP-A-0 188978 et EP-A-0 190 530 et les complexes du platine et d'organopolysiloxane vinylé décrits dans les brevets américains US-A- 3 419 593, US-A- 3 377 432 et US-A- 3 814 730. Pour faire réagir les composés de formule (1bis) ou (2bis) avec le composé de formule (4bis), on utilise généralement une quantité de catalyseur au platine, calculée en poids de platine métal, comprise entre 5 et 600 ppm, de préférence entre 10 et 200 ppm, basée sur le poids de composés de formule (1bis) ou (2bis). La réaction d'hydrosylilation peut avoir lieu en masse ou au sein d'un solvant organique volatil tel que le toluène, l'heptane, le xylène, le tétrahydrofuranne et le tétrachloréthylène. Il est généralement souhaitable de chauffer le mélange réactionnel à une température comprise entre 60 et 120°C pendant le temps pour que la réaction soit complète. On peut ajouter goutte à goutte le composé de formule (1bis) ou (2bis) sur le composé de formule (4bis) en solution dans un solvant organique contenant le catalyseur. On peut aussi ajouter simultanément le composé de formule (1bis) ou (2bis) et le composé de formule (4bis) à une suspension de catalyseur dans un solvant organique. Il est recommandé de vérifier que la réaction est complète en dosant les SiH résiduels par la potasse alcoolique, puis on élimine le solvant, par exemple par distillation sous pression réduite. L'huile brute obtenue peut être purifiée, par exemple par passage sur une colonne absorbante de silice.

Concernant la préparation des filtres de type triorganosilanes de formule (3) donnée précédemment, on peut procéder comme indiqué ci-dessus, toujours par réaction d'hydrosylilation, entre un silane de départ de formule $(R')_3$Si-H (formule (3bis), dans laquelle R' a la même signification que pour le composé de formule (3), et un dérivé organique d'oxanilide de formule (4bis) définie ci-avant.

Comme indiqué précédemment, les composés de formules (1) à (3) ci-dessus présentent un excellent pouvoir filtrant intrinsèque à l'égard du rayonnement ultraviolet (UV-A ou UV-B, selon la structure du produit), ainsi qu'une excellente photostabilité. En mélangeant des produits de structure différente, c'est à dire plus précisément en mélangeant des produits conformes à l'invention présentant une activité purement UV-A avec des produits conformes à l'invention présentant une activité purement UV-B, il est ainsi possible de disposer d'une composition qui présentera globalement une activité filtrante dans toute la gamme des UV nocifs (UV-A + UV-B), ce qui constitue un avantage important. En outre, de par leur caractère fortement liposoluble, les composés de formule (1) à (3) ci-dessus peuvent être utilisés à de grandes concentrations, ce qui confère aux compositions finales des indices de protection très élevés ; par ailleurs, ils se répartissent uniformément dans les supports cosmétiques classiques contenant au moins une phase grasse ou un solvant organique cosmétiquement acceptable et peuvent être ainsi appliqués sur la peau ou les cheveux pour constituer un film protecteur efficace. Enfin, leurs propriétés cosmétiques sont très bonnes, à savoir en particulier que ces produits, par rapport aux silicones filtres de l'art antérieur, sont moins collantes et apportent plus de douceur.

La présente invention a donc aussi pour objet une composition cosmétique comprenant, dans un support cosmétiquement acceptable contenant de préférence au moins une phase grasse ou un solvant organique, une quantité efficace d'au moins un composé de formules (1) à (3) définies ci-avant.

Les composés de formules (1) à (3) sont généralement présents dans des proportions comprises entre 0,1 % et 20 % en poids, de préférence entre 0,5 % et 10 % en poids, par rapport au poids total de la composition.

La composition cosmétique de l'invention peut être utilisée comme composition protectrice de l'épiderme humain ou des cheveux contre les rayons ultraviolets, comme composition antisolaire ou comme produit de maquillage.

Cette composition peut se présenter en particulier sous forme de lotion, de lotion épaissie, de gel, de crème, de lait, de poudre, de bâtonnet solide et eventuellement être conditionnée en aérosol et se présenter sous forme de mousse ou de spray.

Elle peut contenir les adjuvants cosmétiques habituellement utilisés dans le domaine, tels que des corps gras, des solvants organiques, des silicones, des épaississants, des adoucissants, des filtres solaires complémentaires, des agents anti-mousses, des agents hydratants, des parfums, des conservateurs, des tensio-actifs, des charges, des sequestrants, des polymères anioniques, cationiques, non ioniques ou amphotères ou leurs mélanges, des propulseurs, des agents alcalinisants ou acidifiants, des colorants, des pigments ou nanopigments en particulier ceux destinés à assurer un effet photoprotecteur complémentaire par blocage physique du rayonnement ultraviolet, ou tout autre ingrédient habituellement utilisé en cosmétique, en particulier pour la fabrication des compositions antisolaires.

Parmi les solvants organiques, on peut citer les alcools et polyols inférieurs tels que l'éthanol, l'isopropanol, le propylèneglycol, la glycérine et le sorbitol. Les corps gras peuvent être constitués par une huile ou par une cire ou leurs mélanges, des acides gras, des esters d'acides gras, des alcools gras, la vaseline, la paraffine, la lanoline, la lanolinehydrogénée, la lanoline acétylée. Les huiles peuvent être choisies parmi les huiles animales, végétales, minérales ou de synthèse, et notamment l'huile de palme hydrogénée, l'huile de ricin hydrogénée, l'huile de vaseline, l'huile de paraffine, l'huile de Purcellin, les huiles de silicones, volatiles ou non, et les isoparaffines.

Lorsque la composition cosmétique selon l'invention est plus particulièrement destinée à la protection de l'épiderme humain contre les rayons UV ou comme composition antisolaire, elle peut se présenter sous forme de suspension ou de dispersion dans des solvants ou des corps gras, ou encore sous forme d'émulsion (notamment de type H/E ou E/H, mais de préférence H/E) telle qu'une crème ou un lait, de dispersion vésiculaire, sous forme de pommade, de gel, de bâtonnet solide ou de mousse aérosol. Les émulsions peuvent contenir en outre des agents tensio-actifs anioniques, non ioniques, cationiques ou amphotères. Lorsque la composition cosmétique selon l'invention est utilisée pour la protection des cheveux, elle peut se présenter sous forme de shampooing, de lotion, de gel ou composition à rincer, à appliquer avant ou après shampooing, avant ou après coloration ou décoloration, avant, pendant ou après permanente ou défrisage, de lotion ou gel coiffant ou traitant, de lotion ou gel pour le brushing ou la mise en plis, de laque pour cheveux, de composition de permanente ou de défrisage, de coloration ou décoloration des cheveux.

Lorsque la composition cosmétique selon l'invention est utilisée comme produit de maquillage des cils, des sourcils, de la peau ou des cheveux, tels que crème de traitement de l'épiderme, fond de teint, bâton de rouge à lèvres, fards à paupière, fards à joues, ligneur encore appelé "eye-liner", mascara, gel colorant, elle peut se présenter sous forme solide ou pâteuse, anhydre ou aqueuse, comme des émulsions huile-dans-eau ou eau-dans-huile, des suspensions ou encore des gels.

L'invention a aussi pour objet un procédé non thérapeutique de protection de la peau et cheveux contre le rayonnement ultraviolet, en particulier le rayonnement solaire, qui consiste à appliquer sur la peau ou les cheveux une quantité efficace de la composition cosmétique définie ci-dessus, ou d'un composé de formule (1), (2) ou (3) tel que défini ci-avant.

Les exemples qui suivent illustrent l'invention sans toutefois en limiter la portée.

**EXEMPLE 1**

Cet exemple illuste la préparation du N,N'-Bis-{4-[3-[1,3,3,3-tetraméthyl-1-[(triméthylsilyl)oxy]disiloxanyl](2-méthylpropyloxy)-phényl]}-oxalamide, c'est à dire d'un composé conforme à l'invention répondant à la formule :

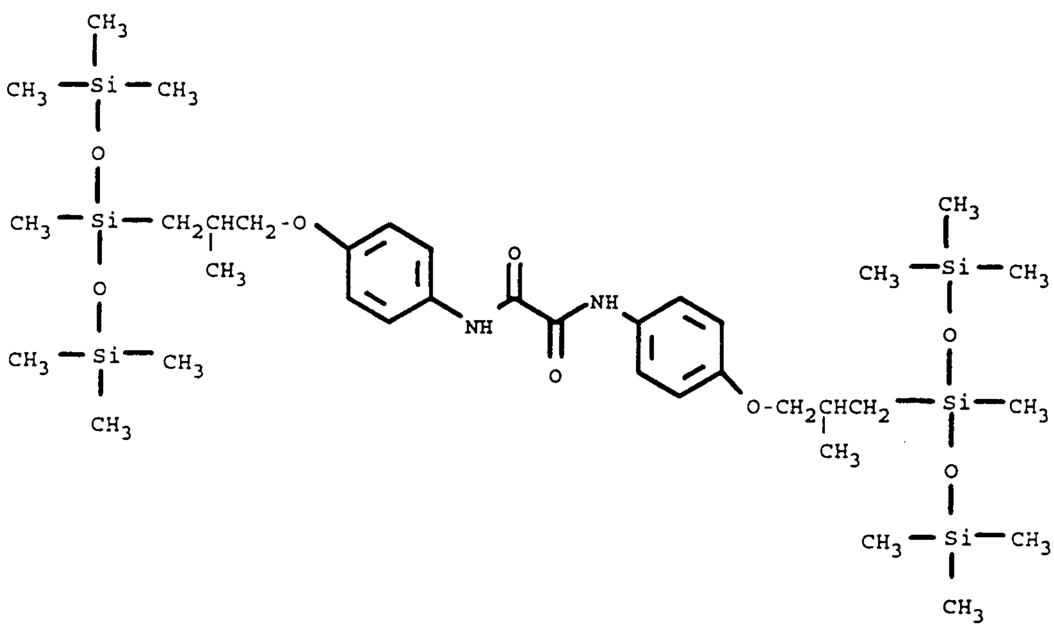

(ce produit correspond à un composé de formule (1) pour lequel R = B = méthyle; r = 0, s = 1 ; q = 0; X = Z = -Y- (en position para) avec pour Y : m = p = 1 et $R^2$ = méthyle

a) **Première étape** :

Dans un ballon tout équipé, on place du para amino phénol (109 g ; 1 mole), du diméthyl oxalate (59 g ; 0,5 mole) et 500 ml d'ortho dichloro benzène. On chauffe le tout à 140°C pendant 4 heures, distille le méthanol formé et poursuit par une heure de chauffage à 170°C. Après refroidissement, le mélange réactionnel est filtré ; la pâte bleue ainsi obtenue est lavée avec du toluène puis de l'éthanol et séchée sous vide. On obtient alors 61 g (Rendement : 44%) de N,N'-Bis-(4-hydroxy-phényl)-oxalamide, de caractéristiques suivantes :

Poudre gris violet
Pf :> 250°C

b) **Deuxième étape** :

Dans un ballon tout équipé, on charge 27,2 g du produit obtenu précédemment, 200 ml de DMSO sec et 28 g de carbonate de potassium. Le mélange est porté à 65°C sous azote. On y ajoute, goutte à goutte et en 20 minutes, du chlorure de méthallyle (20 g ; 0.22 mole). On laisse le tout sous agitation à 65°C pendant 6 heures sous azote. On refroidit, et on ajoute 200 ml de méthanol. Le solide obtenu est filtré, lavé au méthanol et recristallisé dans 400 ml de DMF. On récupère ainsi 14,3 g de N,N'-Bis-[4-(2-méthyl-allyloxy)-phényl]-oxalamide, de caractéristiques suivantes :

Poudre blanche
Pf : 239-240°C

c) **Troisième étape** :

Dans un ballon tout équipé, on place 7,6 g (soit 0,02 mole) du produit obtenu précédemment et 16 ml de toluène. Le mélange est porté à 80°C sous azote. On ajoute ensuite le catalyseur d'hydrosylilation (complexe à 3-3,5% de Pt dans du cyclovinylméthylsiloxane de Hüls Petrarch PC085 : 100 μl) puis 9,79 g d'heptaméthyltrisiloxane. Après 6 heures à 80°C sous azote, on concentre le milieu réactionnel et on effectue une chromatographie sur silice sous pression (éluant : $CH_2Cl_2$/heptane 60:40). On récupère ainsi 16,5 g (Rendement : 78%) du produit final désiré, de caractéristiques suivantes :

Poudre blanche
Pf : 84-86°C.

| Analyse élémentaire : | | | | |
|---|---|---|---|---|
| théorie : | C52,38 | H8,30 | N3,39 | Si20,41 |
| trouvé : | C52,58 | H8,34 | N3,37 | Si19,93 |

Les caractéristiques en absorption UV (mesurée dans l'éthanol) de ce produit sont les suivantes :

$$\lambda_{max} = 293 \text{ nm}, \qquad \varepsilon_{max} = 23000$$

Ce produit peut donc être utilisé très efficacement à titre de filtre solaire actif dans l'UV- B.

**EXEMPLE 2**

Cet exemple illuste la préparation du N,N'-Bis-{2-[3-[1,3,3,3-tetraméthyl-1-[(triméthylsilyl)oxy]disiloxanyl](2-méthyl-propyloxy)-phényl]}-oxalamide, c'est à dire d'un autre composé conforme à l'invention répondant à la formule :

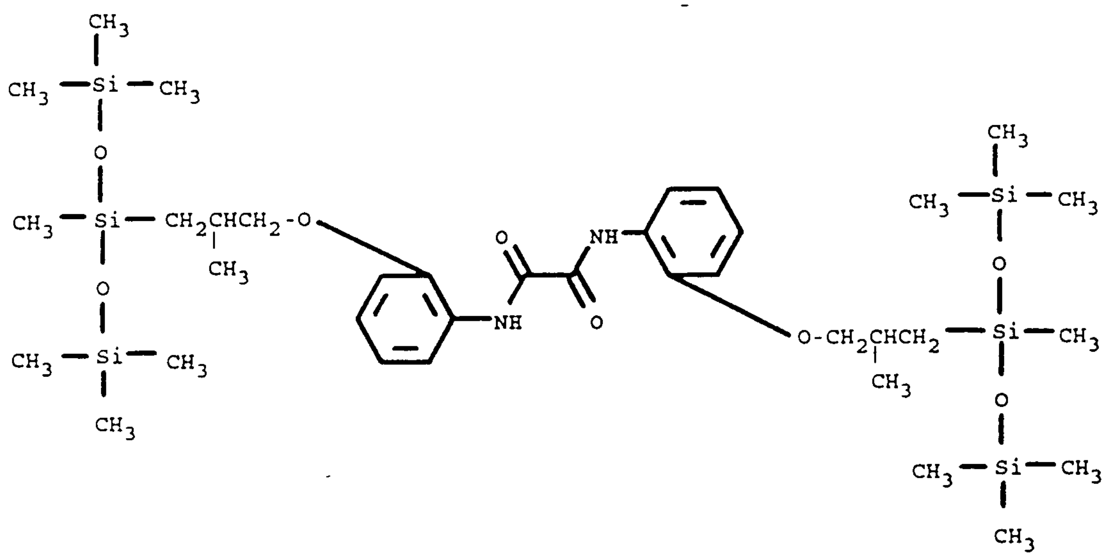

(ce produit correspond à un composé de formule (1) pour lequel R = B = méthyle; r = 0 , s = 1; q = 0; X = Z = -Y- (en position ortho) avec pour Y : m = p = 1 et $R^2$ = méthyle

a) **Première étape** :

Dans un ballon tout équipé, on place de l' ortho amino phénol (65,5 g ; 0.6 mole), du diméthyl oxalate (35,4 g ; 0,3 mole) et 300 ml de 1,2-dichloro benzène. On chauffe le tout à 140°C pendant 4 heures, distille le méthanol formé et poursuit par une heure de chauffage à 170°C. Après refroidissement, le mélange réactionnel est filtré. Le solide obtenu est lavé avec de l'éthanol. On récupère ainsi 69,8g (Rendement : 85%) de N,N'-Bis-(2-hydroxy-phényl)-oxalamide, de caractéristiques suivantes :

Poudre beige clair
Pf :> 250°C

b) **Deuxième étape** :

Dans un ballon tout équipé, on charge 59,8 g du produit obtenu précédemment, 440 ml de DMSO sec et 61,6 g de carbonate de potassium. Le mélange est ensuite porté à 65°C sous azote. Puis, on y ajoute goutte à goutte et en 30 minutes du chlorure de méthallyle (43,8 g ; 0,484 mole). On laisse ensuite le tout sous agitation à 65°C pendant 4 heures sous azote. On refroidit et ajoute 200 ml de méthanol. Le solide obtenu est filtré, lavé au méthanol, à l' eau puis au méthanol à nouveau. On obtient ainsi 77,4 g de N,N'-Bis-[2-(2-méthyl-allyloxy)-phényl]-oxalamide, de caractéristiques suivantes :

Poudre légèrement beige
Pf : 184 °C

c) **Troisième étape** :

Dans un ballon tout équipé, on place 19 g (soit 0,05 mole) du produit obtenu précédemment et 30 ml de toluène. Le mélange est porté à 80°C sous azote. On ajoute ensuite le catalyseur d'hydrosylilation (complexe à 3-3,5% de Pt dans du cyclovinylméthylsiloxane de Hüls Petrarch PC085 : 100 µl), puis 24,5 g d'heptaméthyltrisiloxane. Après 3 heures au reflux sous azote, on concentre le milieu réactionnel. Après recristallisation dans du méthanol, on récupère 28 g (Rendement : 68 %) du produit final désiré, de caractéristiques suivantes :

Poudre blanche
Pf : 77 °C.

| Analyse élémentaire : | | | | |
|---|---|---|---|---|
| théorie : | C52,38 | H8,30 | N3,39 | Si20,41 |
| trouvé : | C52,36 | H8,27 | N3,56 | Si20,15 |

Les caractéristiques en absorption UV (mesurée dans l'éthanol) de ce produit sont les suivantes :

$\lambda_{max}$ = 307 nm, $\varepsilon_{max}$ = 18 425

Ce produit peut donc être utilisé très efficacement à titre de filtre solaire actif dans l'UV- B.

**EXEMPLE 3**

On illustre ici une formulation concrète de composition cosmétique antisolaire conforme à l'invention, à savoir une crème antisolaire de type huile-dans- eau.

| | |
|---|---|
| - Composé de l'exemple 1 | 3 g |
| - Mélange d'alcool cétylstéarylique et d'alcool cétylstéarylique oxyéthyléné à 33 moles d'OE ("DEHSCONET 390" de chez TENSIA) | 8 g |
| - Mélange de mono et distéarate de glycérol non autoémulsifiable | 1,5 g |
| - Alcool cétylique | 1,5 g |
| - Benzoate d'alcools en $C_{12}$-$C_{15}$ ("FINSOLV TN" de chez FINETEX) | 12 g |
| - Polydiméthylsiloxane ("SILBIONE HUILE 70 047 V 300" de chez Rhone-Poulenc) | 1 g |
| - p-méthoxycinnamate de 2-éthyl héxyle (filtre solaire complémentaire vendu sous le nom de "PARSOL MCX" par Givaudan-Roure) | 5 g |
| - Oxyde de titane de qualité nanopigmentaire ("MT 100 T" de chez TAYCA) | 1 g |
| - Glycérine | 15 g |
| - Acide benzène 1,4-di(3-méthylidène-10-camphosulfonique) (filtre solaire complémentaire, décrit dans FR-2 528 420) | 6,06 g |
| - Triéthanolamine | 1,2 g |
| - Parfum, conservateur qs | |
| - Eau qsp | 100 g |

Cette crème est préparée selon les techniques classiques de préparation d'émulsions en dissolvant les filtres liposolubles dans la phase grasse contenant les émulsionnants, en chauffant cette phase grasse vers 70-80°C et en ajoutant, sous vive agitation, l'eau chauffée à la même température. On maintient l'agitation pendant 10 à 15 minutes, puis on laisse refroidir sous agitation modérée, et vers 40 °C on ajoute enfin parfum et conservateur.

**EXEMPLE 4**

On illustre ici une autre formulation concrète de composition cosmétique antisolaire conforme à l'invention, à savoir un lait antisolaire de type huile-dans-eau.

| | |
|---|---|
| - Composé de l'exemple 1 | 2 g |
| - Mélange d'alcool cétylstéarylique et d'alcool cétylstéarylique oxyéthyléné à 33 moles d'OE ("DEHSCONET 390" de chez TENSIA) | 3 g |
| - Mélange de mono et distéarate de glycérol non autoémulsifiable | 1 g |
| - Alcool cétylique | 1 g |
| - Benzoate d'alcools en $C_{12}$-$C_{15}$ ("FINSOLV TN" de chez FINETEX) | 9 g |
| - Polydiméthylsiloxane ("SILBIONE HUILE 70 047 V 300" de chez Rhone-Poulenc) | 1 g |
| - 2-cyano 3,3-diphénylacrylate de 2-éthyl héxyle (filtre solaire complémentaire vendu sous le nom de "UVINUL N 539" de chez BASF) | 6 g |
| - 4-tertiobutyl 4'-méthoxy dibenzoylméthane (filtre solaire complémentaire vendu sous le nom de "PARSOL 1789" par Givaudan-Roure) | 2 g |
| - Glycérine | 15 g |
| - Parfum, conservateur qs | |
| - Eau qsp | 100 g |

Cette crème est préparée selon les techniques classiques de préparation d'émulsions en dissolvant les filtres liposolubles dans la phase grasse contenant les émulsionnants, en chauffant cette phase grasse vers 70-80°C et en ajoutant, sous vive agitation, l'eau chauffée à la même température. On maintient l'agitation pendant 10 à 15 minutes, puis on laisse refroidir sous agitation modérée, et vers 40 °C on ajoute enfin parfum et conservateur.

**Revendications**

1. composés de formules (1) à (3) suivantes :

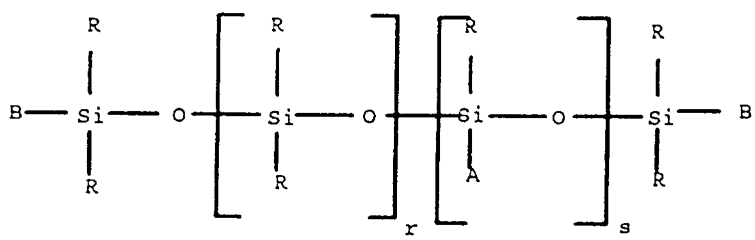

(1)

ou

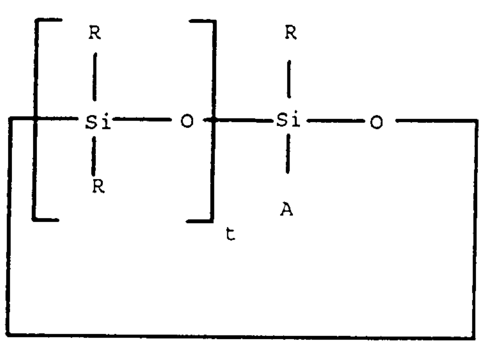

(2)

ou

$$A\text{-}Si(R')_3 \quad (3)$$

formules (1) à (3) dans lesquelles :

- R, identiques ou différents, sont choisis parmi les radicaux alkyles en $C_1$-$C_{10}$, phényle et trifluoro-3,3,3 propyle, au moins 80% en nombre des radicaux R étant méthyle,
- B, identiques ou différents, sont choisis parmi les radicaux R ci-dessus et le radical A défini ci-après,
- R', identiques ou différents, sont choisis parmi les radicaux alkyles en $C_1$-$C_8$, ou phényle,
- r est un nombre entier compris entre 0 et 50 inclusivement, et s est un nombre entier égal à 0 ou 1, avec la condition que si s est nul alors au moins l'un des deux symboles B désigne A,
- t est un nombre entier compris entre 2 et 10 inclusivement,
- et le symbole A désigne un radical lié directement à un atome de silicium, et qui répond à la formule (4) suivante :

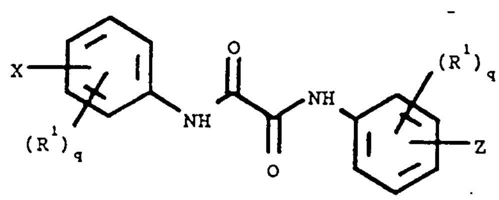

(4)

formule (4) dans laquelle :

* **X** représente un radical divalent -Y- de formule (5) suivante :

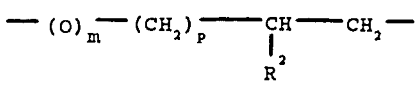

(5)

formule (5) dans laquelle :

- $R^2$ désigne un atome d'hydrogène ou un radical alkyle en $C_1$-$C_4$,
- p est un nombre entier compris entre 0 et 10 inclusivement,
- m est 0 ou 1
- le groupement $-CH_2-$ terminal est directement lié à un atome de silicium ;

* **Z** représente un atome d'hydrogène ou un radical divalent -Y-,

* **q** est un nombre entier compris inclusivement entre 0 et 3,

* $\mathbf{R^1}$, identiques ou différents, sont choisis parmi les radicaux alkyles en $C_1$-$C_8$ ou les radicaux alcoxy en $C_1$-$C_6$, étant entendu que dans ce dernier cas deux $R^1$ adjacents ($q \geq 2$) peuvent former ensemble un groupement alkylidène dioxy dans lequel le groupe alkylidène contient 1 ou 2 atomes de carbone.

**2.** composés selon la revendication 1, répondant à la formule (1) ou à la formule (2), caractérisés par le fait que les radicaux R sont des radicaux alkyle.

**3.** composés selon la revendication 2, caractérisés par le fait que les radicaux R sont des radicaux méthyle, éthyle, propyle, n-butyle, n-octyle ou éthyl-2 hexyle.

**4.** composés selon la revendication 3, caractérisés par le fait que les radicaux R sont des radicaux méthyle.

**5.** composés selon l'une quelconque des revendications précédentes, répondant à la formule (1), caractérisés par le fait que les radicaux B sont des radicaux alkyle.

6. composés selon la revendication 5, caractérisés par le fait que les radicaux B sont des radicaux méthyle, éthyle, propyle, n-butyle, n-octyle ou éthyl-2 hexyle.

7. composés selon la revendication 6, caractérisés par le fait que les radicaux B sont radicaux méthyle.

8. composés selon l'une quelconque des revendications précédentes, répondant à la formule (1), caractérisés par le fait que r est compris entre 0 et 3 inclusivement.

9. composés selon l'une quelconque des revendications 1 à 4, répondant à la formule (2), caractérisés par le fait que t est compris entre 2 et 4 inclusivement.

10. composés selon la revendication 1, répondant à la formule (3), caractérisés par le fait que les radicaux R' sont des radicaux alkyle choisis parmi les radicaux méthyle, éthyle, propyle, n-butyle, n-octyle ou éthyl-2 hexyle.

11. composés selon la revendication 10, caractérisés par le fait que les radicaux R' sont des radicaux méthyle.

12. composés selon l'une quelconque des revendications précédentes, caractérisés par le fait que X est en position ortho ou para du cycle aromatique qui le porte.

13. composés selon l'une quelconque des revendications précédentes, caractérisés par le fait que Z représente -Y- et qu'il est en position ortho ou para du cycle aromatique qui le porte.

14. composés selon l'une quelconque des revendications précédentes, caractérisés par le fait que q est non nul, et de préférence égal à 1, et que $R^1$ est alcoxy, de préférence méthoxy.

15. composés selon l'une quelconque des revendications 1 à 13, caractérisés par le fait que q est nul.

16. composés selon l'une quelconque des revendications précédentes, caractérisés par le fait que dans le radical divalent -Y-, p est égal à 0 ou à 1.

17. composés selon l'une quelconque des revendications précédentes, caractérisés par le fait que dans le radical divalent -Y-, $R^2$ représente l'hydrogène ou un radical méthyle.

18. Utilisation non therapeutique des composés de formules (1)-(3) tels que définis à l'une quelconque des revendications précédentes, à titre de filtres solaires actifs dans le domaine des UV-A et/ou UV-B.

19. Composition cosmétique, en particulier pour la filtration des rayons ultraviolets, caractérisée par le fait qu'elle comprend, dans un support cosmétiquement acceptable, une quantité efficace d'au moins un des composés définis à l'une quelconque des revendications 1 à 17.

20. Composition cosmétique selon la revendication 19, caractérisée par le fait que ledit support cosmétiquement acceptable contient au moins une phase grasse ou un solvant organique.

21. Composition cosmétique selon la revendication 20, caractérisée par le fait que ledit support se présente sous la forme d'une émulsion de type huile-dans-eau ou eau-dans-huile, de préférence huile-dans-eau.

22. Composition cosmétique selon l'une des revendications 19 à 22, caractérisée par le fait que la teneur en composé(s) filtrant(s) est comprise entre 0,1 et 20 % en poids par rapport au poids total de la composition.

23. Composition cosmétique selon la revendication 22, caractérisée par le fait que ladite teneur est comprise entre entre 0,5 et 10 % en poids.

24. Procédé de protection non therapeutique de la peau et/ou des cheveux contre le rayonnement ultraviolet, en particulier le rayonnement solaire, caractérisé par le fait qu'il consiste à appliquer sur la peau et/ou les cheveux une quantité efficace d'au moins un composé ou d'au moins une composition tels que définis à l'une quelconque des revendications précédentes.

## Claims

**1.** Compounds of following formulae (1) to (3):

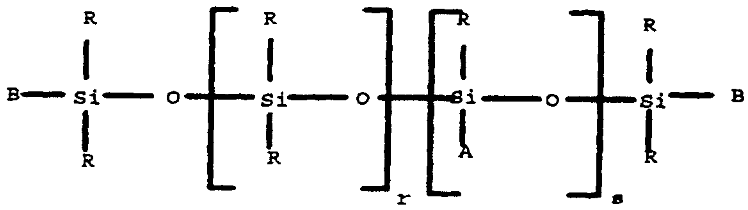

(1)

or

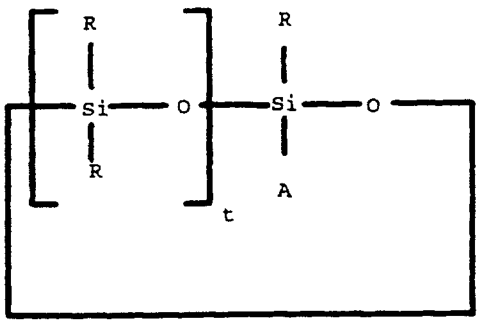

(2)

or

$$A\text{-}Si(R')_3 \qquad (3)$$

in which formulae (1) to (3):

- the groups R, which may be identical or different, are chosen from $C_1$-$C_{10}$ alkyl, phenyl and 3,3,3-trifluoropropyl radicals, at least 80 %, in numerical terms, of the radicals R being methyl,
- the groups B, which may be identical or different, are chosen from the above radicals R and the radical A defined below,
- the groups R', which may be identical or different, are chosen from $C_1$-$C_8$ alkyl radicals or phenyl,
- r is an integer between 0 and 50 inclusively, and s is an integer equal to 0 or 1, with the condition that if s is zero then at least one of the two symbols B denotes A,
- t is an integer between 2 and 10 inclusively,
- and the symbol A denotes a radical directly attached to a silicon atom, and which corresponds to the following formula (4):

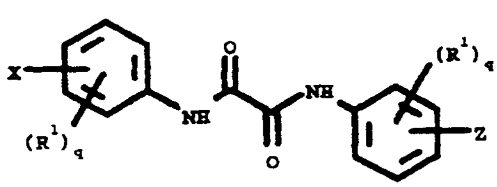

(4)

in which formula (4):

* **X** represents a divalent radical -Y- of following formula (5):

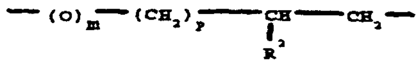 (5)

in which formula (5):

- $R^2$ denotes a hydrogen atom or a $C_1$-$C_4$ alkyl radical,
- p is an integer between 0 and 10 inclusively,
- m is 0 or 1,
- the end $-CH_2-$ group is directly attached to a silicon atom;

* **Z** represents a hydrogen atom or a divalent radical -Y-,
* **q** is an integer between 0 and 3 inclusively,
* the groups $R^1$, which may be identical or different, are chosen from $C_1$-$C_8$ alkyl radicals or $C_1$-$C_6$ alkoxy radicals, it being understood that, in the latter case, two adjacent groups $R^1$ ($q \geq 2$) may together form an alkylidenedioxy group in which the alkylidene group contains 1 or 2 carbon atoms.

**2.** Compounds according to Claim 1, corresponding to formula (1) or to formula (2), characterized in that the radicals R are alkyl radicals.

**3.** Compounds according to Claim 2, characterized in that the radicals R are methyl, ethyl, propyl, n-butyl, n-octyl or 2-ethylhexyl radicals.

**4.** Compounds according to Claim 3, characterized in that the radicals R are methyl radicals.

**5.** Compounds according to any one of the preceding claims, corresponding to formula (1), characterized in that the radicals B are alkyl radicals.

**6.** Compounds according to Claim 5, characterized in that the radicals B are methyl, ethyl, propyl, n-butyl, n-octyl or 2-ethylhexyl radicals.

**7.** Compounds according to Claim 6, characterized in that the radicals B are methyl radicals.

**8.** Compounds according to any one of the preceding claims, corresponding to formula (1), characterized in that r is between 0 and 3 inclusively.

**9.** Compounds according to any one of Claims 1 to 4, corresponding to formula (2), characterized in that t is between 2 and 4 inclusively.

**10.** Compounds according to Claim 1, corresponding to formula (3), characterized in that the radicals R' are alkyl radicals chosen from the methyl, ethyl, propyl, n-butyl, n-octyl and 2-ethylhexyl radicals.

**11.** Compounds according to Claim 10, characterized in that the radicals R' are methyl radicals.

**12.** Compounds according to any one of the preceding claims, characterized in that X is in the ortho or para position on the aromatic ring which bears it.

**13.** Compounds according to any one of the preceding claims, characterized in that Z represents -Y- and in that it is in the ortho or para position on the aromatic ring which bears it.

**14.** Compounds according to any one of the preceding claims, characterized in that q is non-zero, and preferably equal to 1, and in that $R^1$ is alkoxy, preferably methoxy.

**15.** Compounds according to any one of Claims 1 to 13, characterized in that q is zero.

**16.** Compounds according to any one of the preceding claims, characterized in that, in the divalent radical -Y-, p is

equal to 0 or to 1.

17. Compounds according to any one of the preceding claims, characterized in that, in the divalent radical -Y-, $R^2$ represents hydrogen or a methyl radical.

18. Non-therapeutic use of the compounds of formulae (1)-(3) as defined in any one of the preceding claims, as sunscreens which are active in the UV-A and/or UV-B region.

19. Cosmetic composition, in particular for screening out ultraviolet rays, characterized in that it comprises, in a cosmetically acceptable vehicle, an effective amount of at least one of the compounds defined in any one of Claims 1 to 17.

20. Cosmetic composition according to Claim 19, characterized in that the said cosmetically acceptable vehicle contains at least one fatty phase or one organic solvent.

21. Cosmetic composition according to Claim 20, characterized in that the said vehicle is in the form of an emulsion of oil-in-water or water-in-oil type, preferably of oil-in-water type.

22. Cosmetic composition according to one of Claims 19 to 22, characterized in that the content of screening compound(s) is between 0.1 and 20 % by weight relative to the total weight of the composition.

23. Cosmetic composition according to Claim 22, characterized in that the said content is between 0.5 and 10 % by weight.

24. Process for the non-therapeutic protection of the skin and/or the hair against ultraviolet radiation, in particular solar radiation, characterized in that it consists in applying to the skin and/or the hair an effective amount of at least one compound or of at least one composition as are defined in any one of the preceding claims.

**Patentansprüche**

1. Verbindungen der folgenden Formeln (1) bis (3):

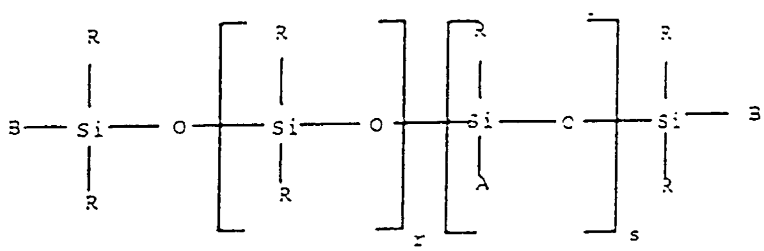

(1)

oder

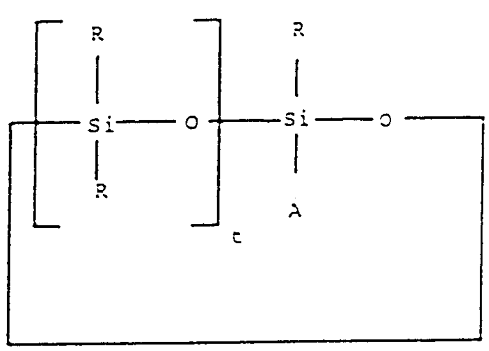

(2)

oder

$$A\text{-}Si(R')_3,$$

wobei in den Formeln (1) bis (3):

- die Gruppen R, die identisch oder voneinander verschieden sind, unter den $C_{1-10}$-Alkylgruppen, Phenyl und 3,3,3-Trifluorpropyl ausgewählt sind, wobei mindestens 80 % der Anzahl der Gruppen R Methyl bedeutet,
- die Gruppen B, die identisch oder voneinander verschieden sind, unter den oben genannten Gruppen R und der nachfolgend definierten Gruppe A ausgewählt sind,
- die Gruppen R', die identisch oder voneinander verschieden sind, unter den $C_{1-8}$-Alkylgruppen oder Phenyl ausgewählt sind,
- r Null oder eine ganze Zahl im Bereich von 1 bis einschließlich 50 und s Null oder 1 bedeutet, mit der Maßgabe, daß mindestens eine der beiden Gruppen B A bedeutet, wenn s Null ist,
- t eine ganze Zahl im Bereich von 2 bis einschließlich 10 bedeutet, und
- die Gruppe A eine direkt an ein Siliciumatom gebundene Gruppe bedeutet, die der folgenden Formel (4) entspricht:

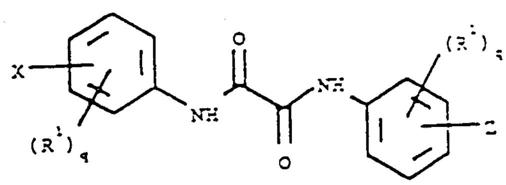

(4) ,

wobei in der Formel (4) bedeuten:

- X eine zweiwertige Gruppe -Y- der folgenden Formel (5):

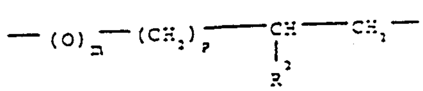

(5) ,

wobei in der Formel (5) bedeuten:

  - $R^2$ Wasserstoff oder $C_{1-4}$-Alkyl,
  - p Null oder eine ganze Zahl von 1 bis einschließlich 10,
  - m Null oder 1, und
  - die Endgruppe $-CH_2-$ ist direkt an ein Siliciumatom gebunden ist;

- Z Wasserstoff oder eine zweiwertige Gruppe -Y-,
- q Null, 1, 2 oder 3,
- die Gruppen $R^1$, die identisch oder voneinander verschieden sind, $C_{1-8}$-Alkyl oder $C_{1-6}$-Alkoxy, mit der Maßgabe, daß im letzten Fall zwei benachbarte Gruppe $R^1$ ($q \geq 2$) gemeinsam eine Alkylidendioxygruppe bilden können, wobei die Alkylidengruppe 1 oder 2 Kohlenstoffatome enthält.

**2.** Verbindungen nach Anspruch 1, die der Formel (1) oder der Formel (2) entsprechen, dadurch gekennzeichnet, daß die Gruppen R Alkylgruppen sind.

**3.** Verbindungen nach Anspruch 2, dadurch gekennzeichnet, daß die Gruppen R die Gruppen Methyl, Ethyl, Propyl,

n-Butyl, n-Octyl oder 2-Ethylhexyl bedeuten.

4. Verbindungen nach Anspruch 3, dadurch gekennzeichnet, daß die Gruppen R Methyl bedeuten.

5. Verbindungen nach einem der vorhergehenden Ansprüche, die der Formel (1) entsprechen, dadurch gekennzeichnet, daß die Gruppen B Alkylgruppen bedeuten.

6. Verbindungen nach Anspruch 5, dadurch gekennzeichnet, daß die Gruppen B die Gruppen Methyl, Ethyl, Propyl, n-Butyl, n-Octyl oder 2-Ethylhexyl bedeuten.

7. Verbindungen nach Anspruch 6, dadurch gekennzeichnet, daß die Gruppen B Methyl bedeuten.

8. Verbindungen nach einem der vorhergehenden Ansprüche, die der Formel (1) entsprechen, dadurch gekennzeichnet, daß r im Bereich von Null bis einschließlich 3 liegt.

9. Verbindungen nach einem der Ansprüche 1 bis 4, die der Formel (2) entsprechen, dadurch gekennzeichnet, daß t im Bereich von 2 bis einschließlich 4 liegt.

10. Verbindungen nach Anspruch 1, die der Formel (3) entsprechen, dadurch gekennzeichnet, daß die Gruppen R' Alkylgruppen sind, die unter Methyl, Ethyl, Propyl, n-Butyl, n-Octyl oder 2-Ethylhexyl ausgewählt sind.

11. Verbindungen nach Anspruch 10, dadurch gekennzeichnet, daß die Gruppen R' Methyl bedeuten.

12. Verbindungen nach einem der vorhergehenden Ansprüche, dadurch gekennzeichnet, daß sich X in o- oder p-Stellung des aromatischen Rings befindet, der die Gruppe trägt.

13. Verbindungen nach einem der vorhergehenden Ansprüche, dadurch gekennzeichnet, daß Z -Y- bedeutet und sich in o- oder p-Stellung des aromatischen Rings befindet, der die Gruppe trägt.

14. Verbindungen nach einem der vorhergehenden Ansprüche, dadurch gekennzeichnet, daß q nicht Null und vorzugsweise 1 ist und $R^1$ Alkoxy und vorzugsweise Methoxy bedeutet.

15. Verbindungen nach einem der Ansprüche 1 bis 13, dadurch gekennzeichnet, daß q Null ist.

16. Verbindungen nach einem der vorhergehenden Ansprüche, dadurch gekennzeichnet, daß p in der zweiwertigen Gruppe -Y- Null oder 1 ist.

17. Verbindungen nach einem der vorhergehenden Ansprüche, dadurch gekennzeichnet, daß $R^2$ in der zweiwertigen Gruppe -Y- Wasserstoff oder Methyl bedeutet.

18. Nichttherapeutische Verwendung der Verbindungen der Formel (1) bis (3) nach einem der vorhergehenden Ansprüche als Sonnenschutzfilter, die im UV-A- und/oder UV-B-Bereich wirksam sind.

19. Kosmetische Zusammensetzung, insbesondere um UV-Strahlung zu filtern, dadurch gekennzeichnet, daß sie in einem kosmetisch akzeptablen Träger eine wirksame Menge mindestens einer der Verbindungen nach einem der Ansprüche 1 bis 17 enthält.

20. Kosmetische Zusammensetzung nach Anspruch 19, dadurch gekennzeichnet, daß der kosmetisch akzeptable Träger mindestens eine Fettphase oder ein organisches Lösungsmittel enthält.

21. Kosmetische Zusammensetzung nach Anspruch 20, dadurch gekennzeichnet, daß der Träger in Form einer Öl-in-Wasser-Emulsion oder Wasser-in-Öl-Emulsion und vorzugsweise als Öl-in-Wasser-Emulsion vorliegt.

22. Kosmetische Zusammensetzung nach einem der Ansprüche 19 bis 22, dadurch gekennzeichnet, daß der Gehalt an Filterverbindung oder Filterverbindungen im Bereich von 0,1 bis 20 Gew.-%, bezogen auf das Gesamtgewicht der Zusammensetzung, liegt.

23. Kosmetische Zusammensetzung nach Anspruch 22, dadurch gekennzeichnet, daß der Gehalt im Bereich von 0,5

bis 10 Gew.-% liegt.

**24.** Nichttherapeutisches Verfahren zum Schutz der Haut und/oder der Haare gegen UV-Strahlung und insbesondere gegen Sonnenlicht, dadurch gekennzeichnet, daß es darin besteht, auf die Haut und/oder die Haare eine wirksame Menge mindestens einer Verbindung oder mindestens einer Zusammensetzung nach einem der vorhergehenden Ansprüche aufzutragen.